# EUROPÄISCHE PATENTSCHRIFT

(11) **EP 0 910 393 B1**
(45) Veröffentlichungstag und Bekanntmachung des Hinweises auf die Patenterteilung: **05.12.2001**
(21) Anmeldenummer: 97918119.5
(22) Anmeldetag: 08.04.1997
(51) Int. Cl.: A61K 33/24, A61K 31/29, A61K 33/04, A61K 45/06

(54) **MITTEL ZUR TOPISCHEN BEHANDLUNG VON HAUTERKRANKUNGEN**
AGENT FOR THE TOPICAL TREATMENT OF SKIN CONDITIONS
AGENT POUR TRAITEMENT TOPIQUE DE MALADIES DE LA PEAU

(30) Priorität: 20.06.1996 DE 19624568
(43) Veröffentlichungstag der Anmeldung: 28.04.1999
(73) Patentinhaber: Lufen, Hans, 47918 Tönisvorst (DE)
(72) Erfinder: Lufen, Hans, 47918 Tönisvorst (DE)
(74) Vertreter: Christophersen, Ruth
(86) Internationale Anmeldenummer: EP9701738
(87) Internationale Veröffentlichungsnummer: WO9748403

(56) Entgegenhaltungen:
- GB-A- 1 038 608
- GB-A- 1 048 820
- US-A- 4 835 148
- DATABASE WPI Week 8609 Derwent Publications Ltd., London, GB; AN 86-055951 XP002038634 & DD 229 029 A (VEB CHEM WERK MILTI) , 30.Oktober 1985

## Beschreibung

Die Erfindung betrifft ein Mittel zur topischen Behandlung von Hauterkrankungen, insbesondere von entzündlichen und hyperproliferativen Hauterkrankungen bzw. kutanen Manifestationen immunologisch bedingter Erkrankungen, sowie die Verwendung dieser Mittel zur topischen Behandlung von Hauterkrankungen.

Die Behandlung von Hauterkrankungen, insbesondere von chronischen Hauterkrankungen, stellt in der Medizin ein großes Problem dar, da sie nur sehr begrenzt heilbar sind. Zu diesen Erkrankungen zählen insbesondere Psoriasis, atopische Dermatitis, Kontakt-Dermatitis und andere ekzematöse Dermatitiden, seborrhoeische Dermatitis, Neurodermitis, Decubitus, Lichen planus, Pemphigus, bulloses Pemphigoid, Epidermolysis bullosa, Urticaria, Angioödeme, Vasculitiden, Erythemen, kutane Eosinophilien, Lupus erythematodes, sowie von Alopecia areata. Die Behandlung dieser Erkrankungen verschafft in vielen Fällen den Patienten nur eine geringe Linderung, in vielen Fällen ist gar kein Heilungserfolg zu beobachten. Hinzukommt, daß eine Vielzahl der eingesetzten Wirkstoffe wie z. B. Kortison starke Nebenwirkungen aufweisen. Die bekannten Mittel schaffen in der Regel nur eine kurzfristige Linderung, eine langfristige Linderung oder sogar Heilung dieser Erkrankungen ist bis jetzt nicht möglich.

Aufgabe der vorliegenden Erfindung ist es, ein Mittel zur topischen Behandlung von Hauterkrankungen zur Verfügung zu stellen, das zumindest eine möglichst langfristige Linderung der auftretenden Symptome, möglichst aber eine Heilung der Erkrankungen ermöglicht.

Gegenstand der vorliegenden Erfindung ist demgemäß ein Mittel zur topischen Behandlung von Hauterkrankungen, enthaltend
A. 0,1 bis 90 Gew.-% Steinkohlenteerlösung,
B. 0,1 bis 40 Gew.-% mindestens einer Komponente aus der Gruppe bestehend aus Talkum, Bismutgallat und feinteiligem Schwefel sowie
C. ggf. einen flüssigen oder festen pharmazeutisch annehmbaren Träger oder Verdünnungsstoff,
wobei sich die Gesamtsumme der einzelnen Komponenten jeweils zu 100 Gew.-% addiert.

Ein weiterer Gegenstand betrifft die Verwendung eines Gemisches, enthaltend
A. 0,1 bis 90 Gew.-% Steinkohlenteerlösung,
B. 0,1 bis 40 Gew.-% mindestens einer Komponente aus der Gruppe bestehend aus Talkum, Bismutgallat und feinteiligem Schwefel sowie
C. ggf. einen flüssigen oder festen pharmazeutisch annehmbaren Träger oder Verdünnungsstoff,
wobei sich die Gesamtsumme der einzelnen Komponenten jeweils zu 100 Gew.-% addiert, zur Herstellung eines Medikaments Behandlung von zur topischen Behandlung von Hauterkrankungen, insbesondere zur Behandlung der oben genannten Hauterkrankungen.

Durch Anwendung des erfindungsgemäßen Mittels ist es möglich, die Symptome von Hauterkrankungen, insbesondere von chronischen Hauterkrankungen, wie entzündlichen und hyperproliferativen Hauterkrankungen bzw. kutanen Manifestationen immunologisch bedingter Erkrankungen, wie Psoriasis, atopischer Dermatitis, Kontakt-Dermatitis und anderen ekzematösen Dermatitiden, seborrhoeischer Dermatitis, Neurodermitis, Decubitus, Lichen planus, Pemphigus, bullosem Pemphigoid, Epidermolysis bullosa, Urticaria, Angioödemen, Vasculitiden, Erythemen, kutanen Eosinophilien, Lupus erythematodes, Ekzeme sowie Alopecia areata langfristig zu lindern und in vielen Fällen sogar zu heilen.

Als Steinkohlenteerlösung der Komponente A kann eine auf dem pharmazeutischen Gebiet handelsübliche Steinkohlenteerlösung eingesetzt werden, die ggf. durch Stabilisatoren, wie Dispergiermittel etc., stabilisiert sein kann, beispielsweise solche, wie sie dem Fachmann auf dem Gebiet der Pharmazie unter der Bezeichnung liqu. carb. deterg. oder liqu. lithan. aceton bekannt sind. Die Menge der Steinkohlenteerlösung ist von der Schwere der Erkrankung und der zu behandelnden Haustelle abhängig. Enthält die eingesetzte Steinkohlenteerlösung große Mengen Lösungsmittel, wie z.B. liqu. lithan. aceton, kann die Komponente A in größeren Mengen eingesetzt werden. Wird das konzentrierte liqu. carb. deterg. eingesetzt, ist die Komponente A im erfindungsgemäßen Mittel vorzugsweise in einer Menge von 0,1 bis 25 Gew.-%, insbesondere von 0,1 bis 20 Gew.-%, enthalten.

Als Komponente B ist mindestens eine Komponente aus der Gruppe bestehend aus Talkum, Bismutgallat und feinteiligem Schwefel enthalten.

Als feinteiliger Schwefel kann auf dem pharmazeutischen Gebiet handelsüblicher Schwefel verwendet werden, der auch unter der Bezeichnung Sulf. praec. bekannt ist.

Als Talkum können, wie auch im Fall der anderen Bestandteile, auf dem pharmazeutischen Gebiet handelsübliche Formen eingesetzt werden, wie z.B. Talk. venet.

Bismutgallat wird vorzugsweise als basisches Bismutgallat, Bismut. subgallic., eingesetzt.

Die Komponente B ist im erfindungsgemäßen Mittel vorzugsweise in einer Menge von 3 bis 30 Gew.-%, insbesondere von 5 bis 25 Gew.-%, enthalten.

Die Bestandteile der Komponente B können im erfindungsgemäßen Mittel einzeln in Mengen jeweils bis zu 40 Gew.-%, vorzugsweise von 1 bis 20 Gew.-%, eingesetzt werden.

Das Mittel kann noch weitere Bestandteile enthalten, die die Wirkung der erfindungsgemäß enthaltenen Komponenten unterstützen. Außerdem kann das Mittel für die jeweilige galenische Form übliche Bestandteile enthalten.

Die Darreichungsformen des erfindungsgemäßen Mittels können die in der pharmazeutischen Industrie üblichen festen und flüssigen Träger- bzw. Verdünnungstoffe enthalten. Bevorzugt werden Träger- und Verdünnungsstoffe, insbesondere bevorzugt sind Wasser, Ethanol, Aceton oder Gemische der voranstehenden. Beispiele von geeigneten galenischen Formen sind Lösungen, Emulsionen, Suspensionen, Lotionen, Gele, Salben oder Cremes, wobei Lösungen, Emulsionen, Suspensionen und Lotionen bevorzugt sind.

Das erfindungsgemäße Mittel kann durch in der pharmazeutischen Industrie für die jeweilige galenische Form übliche Verfahren hergestellt werden. Die erfindungsgemäß enthaltenen Substanzen sowie ggf. weitere Komponenten werden gut vermischt, so daß diese gleichmäßig verteilt sind. Zur Stabilisierung des erhaltenen Mittels können ggf. übliche Stabilisatoren zugesetzt werden.

In einer bevorzugten Ausführungsform enthält das erfindungsgemäße Mittel
A. 0,1 bis 25 Gew.-% Steinkohlenteerlösung, vorzugsweise Liqu. carbon. deterg.,
B. 0,1 bis 20 Gew.-% feinteiligem Schwefel sowie
C. einen üblichen flüssigen Träger oder Verdünnungsstoff, vorzugsweise Ethanol oder ein Gemisch aus Wasser und Ethanol.

In einer weiteren bevorzugten Ausführungsform enthält das erfindungsgemäße Mittel
A. 0,1 bis 90 Gew.-% Steinkohlenteerlösung, vorzugsweise Liqu. lithan. in Aceton
B. 0,1 bis 20 Gew.-% Bismutgallat und 0,1 bis 20 Gew.-% Talkum sowie
C. ggf. einen üblichen flüssigen Träger oder Verdünnungsstoff.

Für die obige Verwendung hängt die zu verabreichende Dosis von der verwendeten Verbindung, der Verabreichungsform sowie der Behandlungsart ab. Es werden sehr gute Ergebnisse erhalten, wenn das Mittel dünn auf die erkrankten Hautstellen aufgetragen wird. In der Regel ist es ausreichend, das Mittel ein- bis zweimal aufzutragen. Sollte nach einem Zeitraum von einigen Tagen bis zu einer Woche noch keine deutliche Linderung eingetreten sein, kann das Mittel nochmals aufgebracht werden.

Die Wirkungen des erfindungsgemäßen Mittels ist aus den folgenden Tests ersichtlich:

### Beispiele

### Herstellungsbeispiele

1. Herstellung einer Emulsion: 7,5 Gew.-% Steinkohlenteerlösung (Liqu. carbon), 7,5 Gew.-% feinteiliger Schwefel (Sulf. praec.) werden vermischt und auf insgesamt 100 Gew.-% eines Ethanol/Wasser-Gemisches (70/30 V/V) aufgefüllt. Das erhaltene Gemisch wird solange gerührt bis eine homogene Emulsion erhalten wird.
2. Herstellung einer Lösung: 85 Gew.-% Steinkohlenteerlösung (Liqu. lithan. aceton), 7,5 Gew.-% Bismut. subgallic. und 7,5 Gew.-% Talkum (Talk. venet.) werden gut verrührt bis ein klare Lösung entsteht.

### Anwendungsbeispiel

### Wirkung bei Neurodermitis

Die oben in Herstellungsbeispiel 1 hergestellte Emulsion wurde bei 4 Probanden, die unter Psoriasis leiden, an 4 aufeinander folgenden Tagen auf die erkrankten Hautstellen aufgetragen. Die Wirkung der erfindungsgemäßen Salbe auf die erkrankte Haut wurde für eine Hautfläche von 10 cm² beobachtet.

| Applikation/Tage | erkrankte Hautfläche/cm² |
|---|---|
| 1 | 10 |
| 2 | 7 |
| 3 | 3 |
| 4 | 1 |
| 5 | 0 |
| (ohne Auftrag des Mittels) | |

Die Erkrankung trat nach einem Zeitraum von 10 Wochen bei keinem der Probanden wieder auf.

Aus dem obigen Beispiel ist ersichtlich, daß das erfindungsgemäße Mittel die Symptome der Hauterkrankungen langfristig lindert bzw. ganz verschwinden läßt.

## Patentansprüche

1. Mittel zur topischen Behandlung von Hauterkrankungen, enthaltend
A. 0,1 bis 90 Gew.-% Steinkohlenteerlösung,
B. 0,1 bis 40 Gew.-% mindestens einer Komponente aus der Gruppe bestehend aus Talkum, Bismutgallat und feinteiligem Schwefel sowie
C. ggf. einen flüssigen oder festen pharmazeutisch annehmbaren Träger oder Verdünnungsstoff,
wobei sich die Gesamtsumme der einzelnen Komponenten jeweils zu 100 Gew.-% addiert.

2. Mittel nach Anspruch 1, **dadurch gekennzeichnet, daß** als Träger Wasser, Ethanol, Aceton oder Gemische der voranstehenden verwendet werden.

3. Mittel nach einem der Ansprüche 1 oder 2, **dadurch gekennzeichnet, daß** es enthält
A. 0,1 bis 25 Gew.-% Sleinkohlenleerlösung, vorzugsweise Liqu. cabon. deterg.,
B. 0,1 bis 20 Gew.-% feinteiligem Schwefel sowie
einen flüssigen Träger oder Verdünnungsstoff, vorzugsweise Ethanol oder ein Gemisch aus Wasser und Ethanol.

4. Mittel nach einem der Ansprüche 1 oder 2, **dadurch gekennzeichnet, daß** es enthält
A. 0,1 bis 90 Gew.-% Steinkohlenteerlösung, vorzugsweise, Liqu. lithan, in aceton
B. 0,1 bis 20 Gew.-% Bismutgallat und 0,1 bis 20 Gew.-% Talkum sowie
C. ggf. einen flüssigen Träger oder Verdünnungsstoff.

5. Verwendung eines Gemisches enthaltend
A. 0,1 bis 90 Gew.-% Steinkohlenteerlösung
B. 0,1 bis 40 Gew.-% mindestens einer Komponente aus der Gruppe bestehend aus Talkum, Bismutgallat und feinteiligem Schwefel sowie
C. einen flüssigen oder festen pharmazeutisch annehmbaren Träger,
wobei sich die Gesamtsumme der einzelnen Komponenten jeweils zu 100 Gew.-% addiert,
zur Herstellung eines Medikaments zur topischen Behandlung von Hauterkrankungen.

6. Verwendung nach Anspruch 5, **dadurch gekennzeichnet, daß** es sich bei den Hauterkrankungen um entzündliche und hyperproliferative Hauterkrankungen bzw. kutanen Manifestationen immunologisch bedingter Erkrankungen, wie Psoriasis, atopische Dermatitis, Kontakt-Dermatitis und anderen ekzematösen Dermatitiden, seborrhoeische Dermatitis, Neurodermitis, Decubitus, Lichen planus, Pemphigus, bullosem Pemphigoid, Epidermolysis bullosa, Urticaria, Angioödemen, Vasculitiden, Erythemen kutanen Eosinophilien, Lupus erythematodes, Ekzeme sowie Alopecia areata handelt.

## Claims

1. Remedy for local treatment of skin diseases, containing
A. 0.1 to 90 % weight coal tar solution
B. 0.1 to 40 % weight of at least one component of the group consisting of talcum, bismuth gallate and fine sulphur, as well as
C. if required, a pharmaceutically approved fluid or solid agent or dilution substance, whereby the total amount of the individual components adds up to 100% weight each.

2. Remedy in accordance with Claim 1 **characterised in that** water, ethanol, acetone or mixed agents of the above are used as carrier.

3. Remedy in accordance with Claims 1 or 2, **characterised in that** it contains
A. 0.1 to 25 % weight coal tar solution, preferably liqu. carbon.deterg.,
B. 0.1 to 20 % weight fine sulphur, as well as
a fluid agent or dilution substance, preferably ethanol or a mixture of water and ethanol.

4. Remedy in accordance with Claims 1 or 2, **characterised in that** it contains
A. 0.1 to 90 % weight coal tar solution, preferably liqu. lithan. in acetone
B. 0.1 to 20 % weight bismuth gallate and 0.1 to 20 % weight talcum, as well as,
C. if required, a fluid agent or dilution substance.

5. Use of a mixture consisting of
A. 0.1 to 90 % weight coal tar solution,
B. 0.1 to 40 % weight at least one of the components from the group consisting of talcum, bismuth gallate and fine sulphur, as well as
C. a pharmaceutically approved fluid or solid agent,
whereby the total amount of individual ingredients adds up to 100% weight each,
for the purpose of the production of medication for localised treatment of skin diseases.

6. Use of Claim 5 indicated by its being related to inflammatory and hyper- proliferate skin diseases, or cutaneous manifestations of immunologically conditioned diseases such as psoriasis, atopic dermatitis, contact dermatitis and other eczematous dermatides, seborrhoeic dermatitis, neurodermitis, decubitus, lichen planus, pemphigus, pemphigoid bulloses, epidermolysis bullosa, urticaria, angioneurotic oedema, vasculitides, erythema, cutaneous eosinophilia, lupus erythematosus, eczema, as well as alopecia areata.

## Revendications

1. Agent pour le traitement topique des maladies de la peau, contentant
A. 0,1 à 90 % en poids de solution de goudron de houille
B. au moins 0,1 à 40 % en poids d'un composant du groupe composé de talc, de gallate de bismuth et des particles fins de soufre ainsi que
C. le cas échéant, un porteur ou un agent de dilution liquide ou solide et pharmaceutiquement acceptable,
la somme totale des composants induviduels s'additionnant respectivement à 100 % en poids.

2. Agent selon la revendication 1, **caractérisé en ce qu'**on utilise comme porteur de l'eau, de l'éthanol, de l'acétone ou des mélanges des substances précitées.

3. Agent selon l'une des revendications 1 ou 2, **caractérisé en ce qu'**il contient
A. 0,1 à 25 % en poids de solution de goudron de houille, de préférence liqu.cabon.deterg.,
B. 0,1 à 20 % en poids des particles fins de soufre ainsi que
un porteur liquide ou un agent de dilution, de préférence de l'éthanol ou un mélange composé d'eau et d'éthanol.

4. Agent selon l'une des revendications 1 ou 2, **caractérisé en ce qu'**il contient
A. 0,1 à 90 % en poids de solution de goudron de houille, de préférence liqu.lithan. dans l'acétone
B. 0,1 à 20 % en poids de gallate de bismuth et 0,1 à 20 % en poids de talc ainsi que
C. le cas échéant, un porteur liquide ou un agent de dilution.

5. Utilisation d'un mélange contenant
A. 0,1 à 90 % en poids de solution de goudron de houille,
B. au moins 0,1 à 40 % en poids d'un composant du groupe composé de talc, de gallate de bismuth et des particles fins de soufre ainsi que
C. un porteur liquide ou solide et pharmaceutiquement acceptable,
la somme totale des composants individuels s'additionnant respectivement à 100 % en poids,
pour la fabrication d'un médicament pour le traitement topique des maladies de la peau.

6. Utiisation selon la revendication 5, **caractérisée en ce que** les maladies de la peau sont des maladies de la peau inflammatoires et hyperprolifératives respectivement des manifestations cutanées des maladies dépendant de l'immunologie, comme le psoriasis, la dermatite atopique, la dermatite de contact et d'autres dermatides eczémateuses, la dermatite séborrhéique, la neurodermatite, le décubitus, le lichen planus, le pemphigus, le pemphigoïde bulleux, l'épidermolysis bullosa, l'urticaire, des angio-oedèmes, des vasculitides, des érythèmes, des éosinophilies cutanées, le lupus erythematodes, des eczèmes ainsi que l'alopecia areata.
